# FASCICULE DE BREVET EUROPEEN

(11) **EP 2 607 457 B1**
(45) Date de publication et mention de la délivrance du brevet: **29.06.2016**
(21) Numéro de dépôt: 12290393.3
(22) Date de dépôt: 14.11.2012
(51) Int. Cl.: C10G 45/62, C10G 45/64, B01J 29/72, B01J 29/74, B01J 29/76, B01J 29/78, C10G 3/00

(54) **PROCÉDÉ DE CONVERSION DE CHARGES PARAFFINIQUES ISSUES DE LA BIOMASSE EN BASES DISTILLATS MOYENS METTANT EN OEUVRE AU MOINS UN CATALYSEUR A BASE DE ZEOLITHE IZM-2**
Umwandlungsverfahren von Paraffingemischen aus einer Biomasse mit Mitteldestillat-Basen, bei dem mindestens ein Katalysator auf Zeolith-IZM-2-Basis zum Einsatz kommt
Method for converting paraffin feedstock from biomass into middle distillate bases using at least one catalyst based on the IZM-2 zeolite

(30) Priorité: 22.12.2011 FR 1104024
(43) Date de publication de la demande: 26.06.2013
(73) Titulaire: IFP Énergies nouvelles, 92852 Rueil-Malmaison Cedex (FR)
(72) Inventeur: Bouchy, Christophe, 69007 Lyon (FR); Guillon Emmanuelle, 69390 Vourles (FR); Marques Mota, Filipe Manuel, 69003 Lyon (FR)

(56) Documents cités:
- FR-A1- 2 931 833
- FR-A1- 2 931 834
- FR-A1- 2 934 793
- FR-A1- 2 934 794
- FR-A1- 2 935 139

## Description

La recherche de nouvelles sources d'énergies renouvelables pour la production de carburants constitue un enjeu majeur. La demande en bases distillats moyens, c'est-à-dire en coupe incorporable au pool kérosène et gazole, est en forte croissance, particulièrement en Europe. L'utilisation de ces nouvelles ressources est un moyen de répondre à cette forte demande, prenant en compte par ailleurs les préoccupations liées à l'environnement

Au sein des différentes voies "alternatives", les bases distillats moyens produites à partir d'une charge paraffinique obtenue à partir d'une charge issue de sources renouvelables, et en particulier d'huiles végétales ou des graisses animales, brutes ou ayant subi un traitement préalable, ainsi que les mélanges de telles charges, présentent des propriétés particulièrement intéressantes. En effet, lesdites charges issues de sources renouvelables contiennent des structures chimiques de type triglycérides ou esters ou acides gras libres, la structure et la longueur de chaîne hydrocarbonée de ces derniers étant compatibles avec les hydrocarbures présents dans les distillats moyens. Lesdites charges issues de sources renouvelables produisent, après hydrotraitement, des charges. paraffiniques, exemptes de composés soufrés et de composés aromatiques.

La demande de brevet n° EP 1,681,337 A décrit la transformation de telles charges par décarboxylation, pour former des paraffines ayant un atome de carbone de moins par rapport aux structures chimiques de départ. L'avantage de cette voie tel que décrit dans ce brevet consiste à limiter la consommation d'hydrogène nécessaire. En revanche, les rendements en bases gazole s'en trouvent réduits. Les catalyseurs utilisés sont des catalyseurs métalliques.

Les brevets US 4,992,605 et US 5,705,722 décrivent des procédés de production de bases pour le pool gazole produites à partir de la transformation directe d'huiles végétales (colza, palme, soja, tournesol) ou de biomasse lignocellulosique en hydrocarbures saturés après hydrotraitement ou hydroraffinage de ces produits seuls.

L'effluent liquide issu de ces procédés d'hydrotraitement est essentiellement constitué de n-paraffines qui peuvent présenter des propriétés de tenue à froid insuffisantes pour être incorporées dans un pool gazole et/ou kérosène. De manière à améliorer les propriétés à froid de cet effluent liquide hydrotraité, une étape d'hydroisomérisation est nécessaire pour transformer les n-paraffines en paraffines branchées présentant de meilleures propriétés à froid. Cette étape d'hydroisomérisation est effectuée sur un catalyseur bifonctionnel présentant à la fois une fonction hydro/déshydrogénante et une fonction acide de Bronsted. Selon le taux d'incorporation et les propriétés à froid visées dans le carburant final, il peut être nécessaire d'effectuer une hydroisomérisation très poussée de l'effluent. Cette étape d'hydroisomérisation s'accompagne généralement de la production de produits de craquage trop légers pour être incorporés dans un pool gazole et/ou kérosène. Il en résulte donc une perte de rendement qu'il est désirable de minimiser.

Les demandes de brevets EP 2 138 553 et EP 2 138 552 décrivent un procédé de traitement d'une charge issue d'une source renouvelable comprenant un hydrotraitement, éventuellement une séparation gaz/liquide, éventuellement suivie d'une élimination des composés azotés, et une hydroisomérisation en présence d'un catalyseur comprenant au moins un métal du groupe VIII et/ou au moins un métal du groupe VIB et au moins un tamis moléculaire zéolithique 10 MR monodimensionnel, de préférence choisi parmi les tamis moléculaires de type structural TON, EUO. ou les tamis moléculaires ZSM-48, ZBM-30, IZM-1, COK-7, EU-2 et EU-11. Lesdits procédés permettent d'obtenir des rendements élevés en base gazole.

Les travaux de recherche effectués par le demandeur l'ont conduit à découvrir que, de façon surprenante, l'utilisation d'un catalyseur à base d'une zéolithe IZM-2 dans un procédé d'hydroconversion d'une charge paraffinique produite à partir de ressources renouvelables, permet d'obtenir de bons rendements en base distillats moyens et en particulier, de limiter la production de produits craqués légers ne pouvant pas être incorporés dans un pool gazole et/ou kérosène.

Un objectif de la présente invention est donc de fournir un catalyseur à base d'IZM-2 très sélectif en hydroisomérisation pour la conversion d'un type de charge paraffinique ayant un nombre d'atomes de carbone compris entre 9 et 25 et produite à partir de ressources renouvelables, permettant de limiter la production de produits craqués légers ne pouvant pas etre incorporés dans un pool gazole et/ou kérosène et donc d'améliorer la sélectivité envers la production en base distillats moyens. FR 2 934 794 divulgue un procédé de production de distillat moyen.

### Objet de l'invention

La présente invention concerne un procédé en continu de conversion d'une charge paraffinique produite à partir de ressources renouvelables en bases distillats moyens (gazole et/ou kerosène).

En particulier, un objet de la présente invention est un procédé de conversion d'une charge paraffinique ayant un nombre d'atomes de carbone compris entre 10 et 22, ladite charge paraffinique étant produite à partir de ressources renouvelables, mettant en oeuvre un catalyseur comprenant au moins un métal hydro-déshydrogénant choisi dans le groupe formé par les métaux du groupe VIB et du groupe VIII de la classification périodique, pris seuls ou en mélange et un support comprenant au moins une zéolithe IZM-2 et au moins un liant, ledit procédé étant effectué à une température comprise entre 150 et 500°C, à une pression comprise entre 0,1 MPa et 15 MPa, à une vitesse spatiale horaire comprise entre 0,1 et 10 h⁻¹ et en présence d'une quantité totale d'hydrogène mélangée à la charge telle que le ratio hydrogène/charge soit compris entre 70 et 2000 Nm³/m³ de charge selon la revendication 1.

Un objet de l'invention est de fournir un procédé de conversion d'une charge paraffinique produite à partir de ressources renouvelables permettant de produire des bases distillats moyens, en particulier une base kérosène et/ou une base gazole, tout en limitant la production de produits légers non incorporables auxdites bases.

Un autre objet de l'invention est d'améliorer le degré de ramification par hydroisomérisation de la charge paraffinique mise en oeuvre et produite à partir de ressources renouvelables, le degré de ramification étant ajusté de manière à obtenir, pour les bases distillats moyens, des propriétés, en particulier à froid, compatibles avec les normes en vigueur pour les distillats moyens.

L'invention présente également l'avantage de fournir un procédé permettant la production de bases distillats moyens correspondant aux nouvelles normes environnementales, à partir de charges issues de sources renouvelables.

Les bases gazole produites sont d'excellente quantité :
- elles présentent une faible teneur en soufre, azote et aromatiques.
- un excellent cétane, en raison de la structure substantiellement paraffinique des hydrocarbures formés.
- des bonnes propriétés de tenue à froid en raison du degré d'isomérisation des paraffines de la coupe;
- une basse densité (généralement moins de 800 kg/m³), ce qui est un avantage dans la mesure où cela facilite l'obtention pour le pool gazole de la spécification en la matière qui est d'au maximum 845 kg/m³.

Les bases kérosène produites sont d'excellente qualité:
- elles présentent une faible teneur en soufre, azote.
- un excellent point de fumée en raison de la faible teneur en aromatiques
- des bonnes propriétés de tenue à froid en raison du degré d'isomérisation des paraffines de la coupe;
- une basse densité (généralement moins de 800 kg/m³).

### Résumé de l'invention.

L'invention porte sur un procédé de conversion d'une charge paraffinique ayant un nombre d'atomes de carbone compris entre 10 et 22, ladite charge paraffinique étant produite à partir de ressources renouvelables, à l'exclusion des charges paraffiniques obtenues par un procédé mettant en jeu une étape de valorisation par la voie Fischer-Tropsch, ledit procédé mettant en oeuvre un catalyseur comprenant au moins un métal hydro-déshydrogénant choisi dans le groupe formé par les métaux du groupe VIB et du groupe VIII de la classification périodique, pris seuls ou en mélange et un support comprenant au moins une zéolithe IZM-2 et au moins un liant, ledit procédé étant effectué à une température comprise entre 150 et 500°C, à une pression comprise entre 0,1 MPa et 15 MPa, à une vitesse spatiale horaire comprise entre 0,1 et 10 h⁻¹ et en présence d'une quantité totale d'hydrogène mélangée à la charge telle que le ratio hydrogène/charge soit compris entre 70 et 2000 Nm³/m³ de charge.

### Description de l'invention

Conformément à l'invention, ladite charge paraffinique ayant un nombre d'atomes de carbone compris entre 10 et 22 utilisée dans le procédé selon l'invention est produite à partir de ressources renouvelables.

Ladite charge paraffinique présente un nombre d'atomes de carbone compris entre 10 et 22.

La teneur en paraffines dans ladite charge mise en oeuvre dans le procédé selon l'invention est avantageusement supérieure à 90% poids, de préférence supérieure à 95% poids, de manière encore plus préférée supérieure à 98% poids.

Conformément à l'invention, ladite charge utilisée dans le procédé selon l'invention est une charge paraffinique produite à partir de ressources renouvelables, à l'exclusion des charges paraffiniques obtenues par un procédé mettant en jeu une étape de valorisation par la voie Fischer-Tropsch. Ainsi, les charges paraffiniques obtenues, selon un procédé de synthèse Fischer-Tropsch, à partir d'un gaz de synthèse (CO+H₂) produit à partir de ressources renouvelables selon la voie BTL aussi appelée selon la terminologie anglo-saxonne "Biomass To Liquid", sont exclues des charges utilisées dans le procédé selon l'invention. De préférence, ladite charge paraffinique est produite à partir de ressources renouvelables choisies parmi les huiles végétales, les huiles d'algues ou algales, les huiles de poissons et les graisses d'origine végétale ou animale, ou des mélanges de telles charges.

Lesdites huiles végétales peuvent avantageusement être brutes ou raffinées, totalement ou en partie, et issues des végétaux choisis parmi le colza, le tournesol, le soja, le palmier, l'olive, la noix de coco, le coprah, le ricin, le coton, les huiles d'arachides, de lin et de crambe et toutes les huiles issues par exemple du tournesol ou du colza par modification génétique ou hybridation, cette liste n'étant pas limitative. Lesdites graisses animales sont avantageusement choisies parmi le lard et les graisses composées de résidus de l'industrie alimentaire ou issus des industries de la restauration. Les huiles de fritures, les huiles animales variées comme les huiles de poisson, le suif, le saindoux peuvent également être utilisées.

Les ressources renouvelables à partir desquelles est produite la charge paraffinique utilisée dans le procédé selon l'invention contiennent essentiellement des structures chimiques de type triglycérides que l'homme du métier connait également sous l'appellation tri ester d'acides gras ainsi que des acides gras libres, dont les chaînes grasses contiennent un nombre d'atomes de carbone compris entre 10 et 22.

La structure et la longueur de chaîne hydrocarbonée de ces derniers est compatible avec les hydrocarbures présents dans le gazole et le kérosène, c'est à dire la coupe distillats moyens. Un tri ester d'acide gras est ainsi composé de trois chaînes d'acides gras. Ces chaînes d'acide gras sous forme de tri ester ou sous forme d'acide gras libres, possèdent un nombre d'insaturations par chaîne, également appelé nombre de doubles liaisons carbone-carbone par chaîne, généralement compris entre 0 et 3 mais qui peut être plus élevé notamment pour les huiles issues d'algues qui présentent généralement un nombre d'insaturations par chaînes de 5 à 6.

Les molécules présentes dans lesdites ressources renouvelables utilisées dans la présente invention présentent donc un nombre d'insaturations, exprimé par molécule de triglycéride, avantageusement compris entre 0 et 18. Dans ces charges, le taux d'insaturation, exprimé en nombre d'insaturations par chaîne grasse hydrocarbonée, est avantageusement compris entre 0 et 6.

Les ressources renouvelables comportent généralement également différentes impuretés et notamment des hétéroatomes tels que l'azote. Les teneurs en azote dans les huiles végétales sont généralement comprises entre 1 ppm et 100 ppm poids environ, selon leur nature. Elles peuvent atteindre jusqu'à 1% poids sur des charges particulières.

Ladite charge paraffinique utilisée dans le procédé selon invention est avantageusement produite à partir de ressources renouvelables selon des procédés connus de l'homme du métier. Une voie possible est la transformation catalytique desdites ressources renouvelables en effluent paraffinique désoxygéné en présence d'hydrogène et en particulier, l'hydrotraitement.

De préférence, ladite charge paraffinique est produite par hydrotraitement desdites ressources renouvelables. Ces procédés d'hydrotraitement de ressources renouvelables sont déjà bien connus et sont décrits dans de nombreux brevets. A titre d'exemple, ladite charge paraffinique utilisée dans le procédé selon l'invention peut avantageusement être produite, de préférence par hydrotraitement puis par séparation gaz/liquide, à partir desdites ressources renouvelables comme dans le brevet FR 2 910 483 ou dans le brevet FR 2 950 895.

Conformément à l'invention, le procédé est un procédé de conversion de ladite charge paraffinique produite à partir de ressources renouvelables mettant en oeuvre un catalyseur comprenant au moins un métal hydro-déshydrogénant choisi dans le groupe formé par les métaux du groupe VIB et du groupe VIII de la classification périodique, pris seuls ou en mélange et un support comprenant au moins une zéolithe IZM-2 et au moins un liant. De préférence, ledit procédé selon l'invention est un procédé d'hydroisomérisation.

Le catalyseur utilisé dans le procédé selon l'invention est avantageusement de type bifonctionnel, c'est-à-dire qu'il possède une fonction hydro/déshydrogénante et une fonction hydroisomérisante.

De préférence, les éléments du groupe VIII sont choisis parmi les métaux nobles et non nobles du groupe VIII et de préférence parmi le fer, le cobalt, le nickel, le ruthénium, le rhodium, le palladium, l'osmium, l'iridium ou le platine, pris seuls ou en mélange et de manière préférée parmi le cobalt, le nickel, le platine et le palladium, pris seul ou en mélange.

Dans le cas où les éléments du groupe VIII sont choisis parmi les métaux nobles du groupe VIII, les éléments du groupe VIII sont avantageusement choisis parmi le platine et le palladium, pris seuls ou en mélange. Dans ce cas, lesdits éléments sont utilisés sous leur forme réduite.

Dans le cas où les éléments du groupe VIII sont choisis parmi les métaux non nobles du groupe VIII, les éléments du groupe VIII sont avantageusement choisis parmi le cobalt et le nickel, pris seuls ou en mélange. De préférence, les éléments du groupe VIB sont choisis parmi le tungstène et le molybdène, pris seuls ou en mélange. Dans le cas où la fonction hydrogénante comprend un élément du groupe VIII et un élément du groupe VIB, les associations de métaux suivants sont préférées: nickel-molybdène, cobalt-molybdène, fer-molybdène, fer-tungstène, nickel-tungstène, cobalt-tungstène, et de manière très préférée: nickel-molybdène, cobalt-molybdène, nickel-tungstène. Il est également possible d'utiliser des associations de trois métaux tel que par exemple nickel-cobalt-molybdène. Lorsqu'une combinaison de métaux du groupe VI et du groupe VIII est utilisée, le catalyseur est alors préférentiellement utilisé sous une forme sulfurée.

Dans le cas où ledit catalyseur comprend au moins un métal noble du groupe VIII, la teneur en métal noble dudit catalyseur est avantageusement comprise entre 0,01 et 5% en poids de manière préférée entre 0,1 et 4% en poids et de manière très préférée entre 0,1 et 2% en poids par rapport à la masse totale dudit catalyseur.

Selon un mode préféré, ledit catalyseur peut également comprendre de l'étain en plus dudit ou desdits métal(aux) noble(s), le teneur en étain étant de préférence comprise entre 0,1 et 0,5% en poids par rapport à la masse totale de catalyseur.

Dans le cas où le catalyseur comprend au moins un métal du groupe VIB en combinaison avec au moins un métal non noble du groupe VIII, la teneur en métal du groupe VIB est avantageusement comprise entre 5 et 40% en poids d'oxyde par rapport à la masse totale dudit catalyseur, de manière préférée entre 10 et 35% en poids d'oxyde et de manière très préférée entre 15 et 30% en poids d'oxyde et la teneur en métal non noble du groupe VIII est avantageusement comprise entre 0,5 et 10% en poids d'oxyde par rapport à la masse totale dudit catalyseur, de manière préférée entre 1 et 8% en poids d'oxyde et de manière très préférée entre 1,5 et 6% en poids d'oxyde.

Conformément à l'invention, ledit catalyseur comprend un support comprenant au moins une zéolithe IZM-2 et au moins un liant.

De préférence, ledit catalyseur comprend de 2 à 80% en poids de zéolithe IZM-2, de manière très préférée de 5 à 50% en poids et de manière encore préférée de 5 à 30% en poids par rapport à la masse totale dudit catalyseur.

La zéolithe IZM-2 est un solide microporeux cristallisé présentant une structure cristalline décrite dans la demande de brevet FR 2 918 050. Le procédé de préparation de la zéolithe IZM-2 est également décrit dans ladite demande.

Ledit solide IZM-2 présente une composition chimique exprimée sur une base anhydre, en termes de moles d'oxydes, définie par la formule générale suivante : XO₂ aY₂O₃ : bM₂/nO. dans laquelle X représente au moins un élément tétravalent, Y représente au moins un élément trivalent et M est au moins un métal alcalin et/ou un métal alcalino-terreux de valence n.

X est préférentiellement choisi parmi le silicium, le germanium, le titane et le mélange d'au moins deux de ces éléments tétravalents, très préférentiellement X est le silicium et Y est préférentiellement choisi parmi l'aluminium, le bore, le fer, l'indium et le gallium, très préférentiellement Y est l'aluminium. M est préférentiellement choisi parmi le lithium, le sodium, le potassium, le calcium, le magnésium et le mélange d'au moins deux de ces métaux et très préférentiellement M est le sodium. De manière préférée, X représente le silicium, le solide cristallisé IZM-2 selon l'invention est alors un solide entièrement silicique lorsque l'élément Y est absent de la composition dudit solide IZM-2. Il est également avantageux d'employer comme élément X un mélange de plusieurs éléments X, en particulier un mélange de silicium avec un autre élément X choisi parmi le germanium et le titane, de préférence le germanium. Ainsi, lorsque le silicium est présent en mélange avec un autre élément X, le solide cristallisé IZM-2 selon l'invention est alors un métallosilicate cristallisé présentant un diagramme de diffraction des rayons X identique à celui décrit dans le tableau 1 lorsqu'il se trouve sous sa forme calcinée. De manière encore plus préférée et en présence d'un élément Y, X étant le silicium et Y étant l'aluminium : le solide cristallisé IZM-2 selon l'invention est alors un aluminosilicate De préférence, la zéolithe IZM-2 est sou forme aluminosilicate.

De préférence, le rapport molaire du nombre d'atomes de silicium sur le nombre d'atomes d'aluminium Si/Alest inférieur à 200, de préférence inférieure à 150, de manière très préférée inférieure à 120.

La zéolithe IZM-2 entrant dans la composition du support du catalyseur selon l'invention est avantageusement échangée par au moins un traitement par une solution d'au moins un sel d'ammonium de manière à obtenir la forme ammonium de la zéolithe IZM-2 qui une fois calcinée conduit à la forme acide (H⁺) de ladite zéolithe IZM-2. Cette étape d'échange peut être effectuée à toute étape de la préparation du catalyseur, c'est-à-dire après l'étape de préparation de la zéolithe IZM-2, après l'étape de mise en forme de la zéolithe IZM-2 par un liant minéral poreux, ou encore après l'étape d'introduction du métal hydro-déshydrogénant. De préférence l'étape d'échange est effectuée après l'étape de mise en forme de la zéolithe IZM-2.

Ladite zéolithe IZM-2 entrant dans la composition du support du catalyseur utilisé dans le procédé selon l'invention est avantageusement au moins en partie, de préférence pratiquement totalement, sous forme acide, c'est-à-dire sous forme acide (H⁺).

Selon invention, le support du catalyseur utilisé dans le procédé selon l'invention contient un liant. Ledit liant peut avantageusement être amorphe ou cristallisé. De préférence, ledit liant est avantageusement choisi dans le groupe formé par l'alumine, la silice, la silice-alumine, les argiles, l'oxyde de titane, l'oxyde de bore et la zircone, pris seuls ou en mélange. On peut choisir également les aluminates. De préférence, ledit liant du support est l'alumine. De manière préférée, ledit liant du support est une matrice contient de l'alumine sous toutes ses formes connues de l'homme du métier, telles que par exemple les alumines de type alpha, gamma, éta, delta. Lesdites alumines diffèrent par leur surface spécifique et leur volume poreux. Ledit liant du support se présente de préférence sous la forme de billes, grains ou extrudés.

De préférence, ledit catalyseur comprend de 5 à 98% en poids de liant, de manière très préférée de 10 à 95% en poids et de manière encore préférée de 20 à 95% en poids par rapport à la masse totale dudit catalyseur.

### Préparation du support IZM-2/liant

### Mise en forme du support IZM2/ liant

Le support du catalyseur utilisé dans le procédé selon l'invention peut avantageusement être préparé selon toutes les méthodes bien connues de l'homme du métier.

Selon un mode de préparation préféré, ladite zéolithe cristallisée IZM-2 peut avantageusement être introduite au cours de la mise en solution ou en suspension des composés d'alumine avantageusement utilisés selon l'invention. Ladite zéolithe cristallisée IZM-2 peut être, sans que cela soit limitatif, par exemple sous forme de poudre, poudre broyée, suspension, suspension ayant subi un traitement de désagglomération. Ainsi, par exemple, ladite zéolithe cristallisée peut avantageusement être mise en suspension acidulée ou non à une concentration ajustée à la teneur finale en solide IZM-2 visée dans le catalyseur utilisé selon la présente invention. Cette suspension appelée couramment une barbotine est alors mélangée avec les composés d'alumine.

Le support du catalyseur utilisé dans le procédé selon l'invention peut avantageusement être mis en forme par toute technique connue de l'homme du métier. La mise en forme peut avantageusement être réalisée par exemple par extrusion, par pastillage, par la méthode de la coagulation en goutte ("oil-drop"), par granulation au plateau tournant ou par toute autre méthode bien connue de l'homme du métier.

La mise en forme peut avantageusement également être réalisée en présence des différents constituants du catalyseur et extrusion de la pâte minérale obtenue, par pastillage, mise en forme sous forme de billes au drageoir tournant ou au tambour, coagulation en goutte, "oil-drop", "oil-up", ou tout autre procédé connu d'agglomération d'une poudre contenant de l'alumine et éventuellement d'autres ingrédients choisis parmi ceux mentionnés plus haut.

Par ailleurs, les supports mis en oeuvre dans le procédé selon la présente invention peuvent avantageusement avoir été traités ainsi qu'il est bien connu de l'homme du métier par des additifs pour faciliter la mise en forme et/ou améliorer les propriétés mécaniques finales des supports. A titre d'exemple d'additifs, on peut citer notamment la cellulose, la carboxyméthyl-cellulose, la carboxy-ethyl-cellulose, du tall-oil, les gommes xanthaniques, des agents tensio-actifs, des agents floculants comme les polyacrytamides, le noir de carbone, les amidons, l'acide stéarique, l'alcool polyacrylique, l'alcool polyvinylique, des biopolymères, le glucose, les polyéthylènes glycols, etc.

On peut avantageusement ajouter ou retirer de l'eau pour ajuster la viscosité de la pâte à extruder. Cette étape peut avantageusement être réalisée à tout stade de l'étape de malaxage.

Pour ajuster la teneur en matière solide de la pâte à extruder afin de la rendre extrudable, on peut avantageusement également ajouter un composé majoritairement solide et de préférence un oxyde ou un hydrate. On utilise de manière préférée un hydrate et de manière encore plus préférée un hydrate d'aluminium. La perte au feu de cet hydrate est avantageusement supérieure à 15%.

L'extrusion peut avantageusement être réalisée par n'importe quel outil conventionnel, disponible commercialement. La pâte issue du malaxage est avantageusement extrudée à travers une filière, par exemple à l'aide d'un piston ou d'une mono-vis ou double vis d'extrusion. Cette étape d'extrusion peut avantageusement être réalisée par toute méthode connue de l'homme de métier.

Les extrudés de support selon l'invention ont avantageusement généralement une résistance à l'écrasement d'au moins 70 N/cm et de manière préférée supérieure ou égale à 100 N/cm.

### Traitement thermique du support IZM-2/liant

Le support du catalyseur mis en oeuvre dans le procédé selon la présente invention est ensuite avantageusement soumis à une étape de séchage.

Ladite étape de séchage est avantageusement effectuée par toute technique connue de l'homme du métier.

De préférence, le séchage est effectué sous flux d'air. Ledit séchage peut également être avantageusement effectué sous flux de tout gaz oxydant, réducteur ou inerte. De préférence, le séchage est avantageusement effectué entre 50 et 180°C, de manière préférée entre 60 et 150°C et de manière très préférée entre 80 et 130°C.

Ledit support, éventuellement séché, subit ensuite de préférence, une étape de calcination.

Ladite étape de calcination est avantageusement réalisée en présence d'oxygène moléculaire, par exemple en effectuant un balayage d'air, à une température avantageusement supérieure à 200°C et inférieure ou égale à 1100°C. Ladite étape de calcination peut avantageusement être effectuée en lit traversé, en lit léché ou en atmosphère statique. Par exemple, le four utilisé peut être un four rotatif tournant ou être un four vertical à couches traversées radiales. De préférence, ladite étape de calcination est effectuée entre plus d'une heure à 200°C à moins d'une heure à 1100°C. La calcination peut avantageusement être opérée en présence de vapeur d'eau et / ou en présence d'une vapeur acide ou basique. Par exemple, la calcination peut être réalisée sous pression partielle d'ammoniaque.

Des traitements post-calcination peuvent éventuellement être effectués, de manière à améliorer les propriétés du support, par exemple texturales.

### Traitements post-synthèse du support IZM-2/liant

Le support IZM-2 / liant du catalyseur mis en oeuvre dans le procédé selon la présente invention peut ainsi être éventuellement soumis à un traitement hydrothermal en atmosphère confinée. On entend par traitement hydrothermal en atmosphère confinée un traitement par passage à l'autoclave en présence d'eau à une température supérieure à la température ambiante.

Au cours de ce traitement hydrothermal, on peut avantageusement traiter le support. Ainsi, on peut avantageusement imprégner le support, préalablement à son passage à l'autoclave, l'autoclavage étant fait soit en phase vapeur, soit en phase liquide, cette phase vapeur ou liquide de l'autoclave pouvant être acide ou non. Cette imprégnation, préalable à l'autoclavage, peut avantageusement être acide ou non. Cette imprégnation, préalable à l'autoclavage peut avantageusement être effectuée à sec ou par immersion du support dans une solution aqueuse acide. Par imprégnation à sec, on entend mise en contact du support avec un volume de solution inférieur ou égal au volume poreux total du support. De préférence, l'imprégnation est réalisée à sec.

L'autoclave est de préférence un autoclave à panier rotatif tel que celui défini dans la demande brevet EP-A-0 387 109.

La température pendant l'autoclavage peut être comprise entre 100 et 250°C pendant une période de temps comprise entre 30 minutes et 3 heures.

### Dépôt de la fonction hydro-déshydragénante

La fonction hydro-déshydrogénante peut avantageusement être introduite à toute étape de la préparation, de manière très préférée après mise en forme dudit support IZM-2 / liant. La mise en forme est avantageusement suivie d'une calcination, la fonction hydro-déshydrogénante peut également avantageusement être introduite avant ou après cette calcination. La préparation se termine généralement par une calcination à une température de 250 à 600°C. Une autre des méthodes préférées selon la présente invention consiste avantageusement à mettre en forme le support IZM-2 / liant après un malaxage de ce dernier, puis passage de la pâte ainsi obtenue au travers d'une filière pour former des extrudés. La fonction hydro-déshydrogénante peut avantageusement être alors introduite en partie seulement ou en totalité, au moment du malaxage. Elle peut également avantageusement être introduite par une ou plusieurs opérations d'échange ionique sur le support calciné.

D'une façon préférée, le support est imprégné par une solution aqueuse. L'imprégnation du support est de préférence effectuée par la méthode d'imprégnation dite "à sec" bien connue de l'homme du métier. L'imprégnation peut avantageusement être effectuée en une seule étape par une solution contenant l'ensemble des éléments constitutifs du catalyseur final.

La fonction hydro-déshydrogénante peut avantageusement être introduite par une ou plusieurs opérations d'imprégnation du support mis en forme et calciné, par une solution contenant au moins un précurseur d'au moins un oxyde d'au moins un métal choisi dans le groupe formé par les métaux du groupes VIII et les métaux du groupe VIB, le(s) précurseur(s) d'au moins un oxyde d'au moins un métal du groupe VIII étant de préférence introduit(s) après ceux du groupe VIB ou en même temps que ces derniers, si le catalyseur contient au moins un métal du groupe VIB et au moins un métal du groupe VIII.

Dans le cas où le catalyseur contient avantageusement au moins un élément du groupe VIB par exemple le molybdène, il est par exemple possible d'imprégner le catalyseur avec une solution contenant au moins un élément du groupe VIB, de sécher, de calciner. L'imprégnation du molybdène peut avantageusement être facilitée par ajout d'acide phosphorique dans les solutions de paramolybdate d'ammonium, ce qui permet d'introduire aussi le phosphore de façon à promouvoir l'activité catalytique.

Les éléments suivants : bore et/ou silicium et/ou phosphore peuvent être introduits dans le catalyseur à tout niveau de la préparation et selon toute technique connue de l'homme du métier.

Une méthode préférée selon l'invention consiste à déposer le ou les éléments promoteurs choisis, par exemple le couple bore-silicium, sur le support IZM-2 mis en forme avec le liant calciné ou non, de préférence calciné. Pour cela on prépare une solution aqueuse d'au moins un sel de bore tel que le biborate d'ammonium ou le pentaborate d'ammonium en milieu alcalin et en présence d'eau oxygénée et on procède à une imprégnation dite à sec, dans laquelle on remplit le volume des pores du précurseur par la solution contenant par exemple le bore. Dans le cas où l'on dépose par exemple également du silicium, on utilise par exemple une solution d'un composé du silicium de type silicone ou émulsion d'huile silicone.

Le ou les élément(s) promoteur(s) choisi(s) dans le groupe formé par le silicium, le bore et le phosphore peuvent avantageusement être introduits par une ou plusieurs opérations d'imprégnation avec excès de solution sur le précurseur calciné.

La source de bore peut avantageusement être l'acide borique, de préférence l'acide orthoborique H₃BO₃, le biborate ou le pentaborate d'ammonium, l'oxyde de bore, les esters boriques. Le bore peut par exemple être introduit sous la forme d'un mélange d'acide borique, d'eau oxygénée et un composé organique basique contenant de l'azote tels que l'ammoniaque, les amines primaires et secondaires, les amines cycliques, les composés de la famille de la pyridine et des quinoléines et les composés de la famille du pyrrole. Le bore peut être introduit par exemple par une solution d'acide borique dans un mélange eau/alcool.

La source de phosphore préférée est l'acide orthophosphorique H₃PO₄, mais ses sels et esters comme les phosphates d'ammonium conviennent également. Le phosphore peut par exemple être introduit sous la forme d'un mélange d'acide phosphorique et un composé organique basique contenant de l'azote tels que l'ammoniaque, les amines primaires et secondaires, les amines cycliques, les composés de la famille de la pyridine et des quinoléines et les composés de la famille du pyrrole.

De nombreuses sources de silicium peuvent avantageusement être employées. Ainsi, on peut utiliser l'orthosilicate d'éthyle Si(OEt)₄, les siloxanes, les polysiloxanes, les silicones, les émulsions de silicones, les silicates d'halogénures comme le fluorosilicate d'ammonium (NH₄)₂SiF₆ ou le fluorosilicate de sodium Na₂SiF₆. L'acide silicomolybdique et ses sels, l'acide silicotungstique et ses sels peuvent également être avantageusement employés. Le silicium peut avantageusement être ajouté par exemple par imprégnation de silicate d'éthyle en solution dans un mélange eau/alcool. Le silicium peut être ajouté par exemple par imprégnation d'un composé du silicium de type silicone ou l'acide silicique mis en suspension dans l'eau.

Les métaux nobles du groupe VIII du catalyseur de la présente invention peuvent avantageusement être présents en totalité ou partiellement sous forme métallique et/ou oxyde.

Les sources d'éléments nobles du groupe VIII qui peuvent avantageusement être utilisées sont bien connues de l'homme du métier. Pour les métaux nobles on utilise les halogénures, par exemple les chlorures, les nitrates, les acides tels que l'acide chloroplatinique, les hydroxydes, les oxychlorures tels que l'oxychlorure ammoniacal de ruthénium. On peut également avantageusement utiliser les complexes cationiques tels que les sels d'ammonium lorsque l'on souhaite déposer le platine sur le solide IZM-2 par échange cationique.

Les catalyseurs ainsi obtenus sont mis en forme sous la forme de grains de différentes formes et dimensions. Ils sont utilisés en général sous la forme d'extrudés cylindriques ou polylobés tels que bilobés, trilobés, polylobés de forme droite ou torsadée, mais peuvent éventuellement être fabriqués et employés sous la forme de poudres concassées, de tablettes, d'anneaux, de billes, de roues. D'autres techniques que l'extrusion, telles que le pastillage ou la dragéification, peuvent avantageusement être utilisées.

De préférence, les catalyseurs mis en oeuvre dans le procédé selon l'invention ont la forme de sphères ou d'extrudés. Il est toutefois avantageux que le catalyseur se présente sous forme d'extrudés d'un diamètre compris entre 0,5 et 5 mm et plus particulièrement entre 0,7 et 2,5 mm. Les formes sont cylindriques (qui peuvent être creuses ou non), cylindriques torsadés, multilobées (2, 3, 4 ou 5 lobes par exemple), anneaux. La forme cylindrique est avantageusement utilisée de manière préférée, mais toute autre forme peut avantageusement être utilisée.

Dans le cas où le catalyseur utilisé dans le procédé selon l'invention comprend au moins un métal noble, le métal noble contenu dans ledit catalyseur doit être réduit. La réduction du métal est avantageusement réalisée par le traitement sous hydrogène à une température comprise entre 150°C et 650°C et une pression totale comprise entre 0,1 et 25 MPa. Par exemple, une réduction consiste en un palier à 150°C de deux heures puis une montée en température jusqu'à 450°C à la vitesse de 1°C/min puis un palier de deux heures à 450°C; durant toute cette étape de réduction, le débit d'hydrogène est de 1000 normaux m³ hydrogène par m³ catalyseur et la pression totale maintenue constante à 0,1 MPa. Toute méthode de réduction ex-situ peut avantageusement être envisagée.

Dans le cas où le catalyseur utilisé dans le procédé selon l'invention comprend au moins un métal du groupe VIB en combinaison avec au moins un métal non noble du groupe VIII, les métaux sont de préférence utilisés sous leur forme sulfurée. La sulfuration du catalyseur peut etre effectuée in situ ou ex situ par toute méthode connue de l'homme du métier.

### Procédé de conversion

La charge paraffinique ayant un nombre d'atomes de carbone compris entre 10 et 22 et produite à partir de ressources renouvelables, est mise en contact, en présence d'hydrogène avec ledit catalyseur, à des températures et des pressions opératoires permettant avantageusement de réaliser une conversion et de préférence une hydroisomérisation permettant d'atteindre les propriétés à froid visée.

Conformément à l'invention, ledit procédé est effectué à une température comprise entre 150 et 500°C, à une pression comprise entre 0,1 MPa et 15 MPa, à une vitesse spatiale horaire comprise entre 0,1 et 10 h-1 et en présence d'une quantité totale d'hydrogène mélangée à la charge telle que le ratio hydrogène/charge soit compris entre 70 et 2000 Nm³/m³ de charge.

De préférence, ledit procédé est effectué à une température comprise entre 150°C et 450°C, et de manière très préférée, entre 200 et 450°C, à une pression comprise entre 0,2 et 15 MPa, de préférence entre 0,5 et 10 MPa et de manière très préférée, entre 1 et 9 MPa, à une vitesse volumique horaire avantageusement comprise entre 0,2 et 7 h⁻¹ et de manière préférée, entre 0,5 et 5 h⁻¹, et en présence d'une quantité totale d'hydrogène mélangée à la charge telle que le ratio hydrogène/charge soit compris entre 100 et 1500 normaux m³ d'hydrogène par m³ de charge et de préférence entre 150 et 1500 normaux m³ d'hydrogène par m³ de charge.

De préférence, l'effluent issu du procédé de conversion selon l'invention est soumis au moins en partie, et de préférence en totalité, à une ou plusieurs séparations. Le but de cette étape est de séparer les gaz du liquide, et notamment, de récupérer les gaz riches en hydrogène pouvant contenir également des légers tels que la coupe C₁ - C₄ et au moins une base gazole et/ou une base kérosène et de préférence une base kérosène.

### Produits obtenus.

Le produit fourni selon ce procédé est doté d'excellentes caractéristiques, qui en font une base gazole d'excellente qualité :
• sa teneur en soufre est inférieure à 10 ppm poids,
▪ sa teneur en aromatiques totaux est inférieure à 5% poids, et la teneur en polyaromatiques inférieure à 2% poids,
▪ l'indice de cétane est excellent, supérieur à 55,
▪ la densité est inférieure à 840 kg/m³, et le plus souvent inférieure à 820 kg/m³,
▪ sa viscosité cinématique à 40°C est 2 à 8 mm²/s,
▪ ses propriétés de tenue à froid sont compatibles avec les normes en vigueur.

La base kérosène obtenue selon le procédé est également d'excellente qualité:
▪ sa teneur en soufre est inférieure à 10 ppm poids,
▪ sa teneur en aromatiques totaux est inférieure à 5% poids, et la teneur en polyaromatiques inférieure à 2% poids,
▪ son point de fumée est supérieur à 21 mm,
▪ ses propriétés à froid sont compatibles avec les normes en vigueur,
▪ la densité est inférieure à 840 kg/m³, et le plus souvent inférieure à 820 kg/m³,

### Exemples

### Exemple 1 : Réparation de la charge paraffinique.

La charge paraffinique est obtenue par hydrotraitement d'une huile de colza de grade DNS (dégommée, neutralisée et séchée). Le catalyseur utilisé pour effectuer l'hydrotraitement est un catalyseur sulfuré NiMoP/alumine comprenant 0,22 % poids de NiO, 21% poids de MoO₃ et de 5% poids de P₂O₅ supporté sur une alumine gamma. Ledit catalyseur ainsi que les conditions opératoires mises en oeuvre pour cette étape d'hydrotraitement sont décrits dans le brevet FR 2 943 071. L'effluent issu de d'hydrotraitement est ensuite envoyé dans un séparateur qui permet de séparer l'eau, l'hydrogène non converti et les gaz formés et en particulier le propane, le CO et le CO₂, l'H₂S, le NH₃ et de produire une charge paraffinique composé à 90% poids de normales paraffines à 17 et 18 atomes de carbone. Une analyse par distillation simulée montre que cette charge paraffinique ne contient pas de molécules bouillant à moins de 150°C.

### Exemple 2 : Préparation du catalyseur d'hydroisomérisation C1 (non conforme).

Le catalyseur C1 est un catalyseur contenant un métal noble et une zéolithe 12 MR tridimensionnelle. Il s'agit d'une zéolithe, USY commerciale, la zéolithe CBV760, fournie par la société ZEOLYST. Cette zéolithe possède un rapport atomique Si/Al (déterminé par Fluorescence X) de 30. Cette zéolithe est malaxée avec un gel d'alumine de type SB3 fourni par la société Condéa-Sasol. La pate malaxée est extrudée au travers d'une filière de 1,4 mm. Après séchage en étuve une nuit à 110°C, les extrudés sont calcinés à 500°C durant deux heures (rampe de montée en température de 5°C/min) en lit traversé sous air sec (2 normaux litres par heure et par gramme de solide). Les extrudés sont ensuite imprégnés à sec par une solution aqueuse de nitrate de platine tétramine Pt(NH₃)₄(NO₃)₂, laissés à maturer en maturateur à eau durant 24 heures à température ambiante puis calcinés à 500°C (rampe de montée en température de 5°C/min) durant deux heures en lit traversé sous air sec (2 normaux litres par heure et par gramme de solide). Les teneurs pondérales de la zéolithe CBV760 et du platine sur le catalyseur fini après calcination sont respectivement de 15% et de 0,32% poids.

### Exemple 3 : Préparation du catalyseur d'hydroisomérisation C2 (non conforme).

Le catalyseur C2 est un catalyseur contenant un métal noble et une zéolithe 10 MR monodimenslonnelle, la ZBM-30. Cette zéolithe est synthétisée conformément au brevet BASF EP-A-46504 avec le structurant organique triéthylènetétramine. La zéolithe brute de synthèse est alors soumise à une calcination à 550°C durant douze heures (rampe de montée en température de 5°C/min) en lit traversé sous air sec (2 normaux litres par heure et par gramme de solide). La zéolithe obtenue présente un rapport atomique Si/Al (déterminé par Fluorescence X) de 45. La mise en forme de la zéolithe avec le gel d'alumine SB3 ainsi que le dépôt de platine sont effectués dans les mêmes conditions que pour le catalyseur C1. Les teneurs pondérales de la zéolithe ZBM30 et du platine sur le catalyseur fini après calcination sont respectivement de 14% et de 0,35% poids.

### Exemple 4 : Réparation du catalyseur d'hydroisomérisation C3 (non conforme).

Le catalyseur C3 est un catalyseur contenant un métal noble et une zéolithe 10 MR monodimensionnelle, la ZSM-22. Cette zéolithe est synthétisée selon la méthode de Ernst et coll. (Applied Catalysis, 1989, 48, 137). La zéolithe brute de synthèse est traitée thermiquement à 400°C durant cinq heures (rampe de montée en température de 5°C/min) en lit traversé sous azote (0.6 normaux litres par heure et par gramme de solide) puis à 550°C pendant seize heures (rampe de montée en température de 5°C/min) sous oxygène (0,6 normaux litres par heure et par gramme de solide). Le solide est ensuite mis sous reflux durant 4 heures dans une solution de chlorure d'ammonium (100 ml de solution par gramme de solide, concentration en chlorure d'ammonium de 0.5 M) afin d'échanger les cations alcalins par des ions ammonium. Finalement le solide est lavé à l'eau distillée afin d'éliminer le chlorure alcalin (test au nitrate d'argent négatif) puis séché une nuit en étuve à 60°C. La zéolithe obtenue présente un rapport atomique Si/Al (déterminé par Fluorescence X) de 30. La mise en forme de la zéolithe avec le gel d'alumine SB3 ainsi que le dépôt de platine sont effectués dans les mêmes conditions que pour le catalyseur C1. Les'teneurs pondérales de la zéolithe ZSM-22 et du platine sur le catalyseur fini après calcination sont respectivement de 18% et de 0,31% poids.

### Exemplé 5 : Préparation du catalyseur d'hyrdroisomérisation C4 (conforme).

Le catalyseur C4 est un catalyseur contenant un métal noble et la zéolithe IZM-2. Cette zéolithe IZM-2 a été synthétisée conformément à l'enseignement de la demande de brevet FR 2 918 050. La zéolithe IZM-2 brute de synthèse subit alors une calcination à 550°C durant dix heures (rampe de montée en température de 5°C/min) en lit traversé sous air sec (2 normaux litres par heure et par gramme de solide). Le solide obtenu est mis sous reflux durant 4 heures dans une solution de nitrate d'ammonium (100 ml de solution par gramme de solide, concentration en chlorure d'ammonium de 10 M) afin d'échanger les cations alcalins par des ions ammonium. Cette étape de mise sous reflux est effectuée quatre fois. Le solide ainsi obtenu possède un rapport Si/Al (déterminé par Fluorescence X) de 53. La mise en forme de la zéolithe avec le gel d'alumine SB3 ainsi que le dépôt de platine sont effectués dans les mêmes conditions que pour le catalyseur C1. Les teneurs pondérales de la zéolithe IZM-2 et du platine sur le catalyseur fini après calcination sont respectivement de 20% et de 0.32% poids.

### Exemple 6 : Hydroisomérisation de la charge paraffinique - production de base gazole.

La charge paraffinique obtenu dans l'exemple 1 est hydroisomérisée sur les différents catalyseurs d'hydroisomérisation en lit traversé dans un réacteur d'hydroisomérisation travaillant en isotherme et à hydrogène perdu. Avant test catalytique, chaque catalyseur subit une étape de réduction sous débit d'hydrogène dans les conditions opératoires suivantes:
- pression totale : 0,1 MPa
- débit d'hydrogène: 1600 normaux litres par heure et par litre de catalyseur
- montée de température ambiante à 120°C à 10°C/minute
- palier d'une heure à 120°C,
- montée de 120°C à 450°C à 5°C/min**,**
- palier de deux heures à 450°C.

La charge paraffinique est hydroisomérisée sur les différents catalyseurs dans les conditions opératoires suivantes:
- pression totale: 5 MPa
- WH (volume de charge / volume de catalyseur / heure): 1 h⁻¹,
- rapport hydrogène / charge: 700 normaux litres / litre,
- température: variable

La température de réaction est ajustée de manière à obtenir une température limite de filtrabilité de la coupe 150°C⁺ de l'effluent hydroisomérisé voisine de -40°C.

Les rendements en coupe 150°C⁻, coupe 150°C⁺ (obtenus par distillation simulée) de l'effluent ainsi que la température limite de filtrabilité (obtenue par la méthode NF EN 116) de la coupe 150°C⁺ de l'effluent sont reportés pour l'ensemble des catalyseurs dans le tableau 1. On constate que pour l'ensemble des catalyseurs il est possible d'obtenir une base gazole présentant d'excellentes propriétés de tenue à froid mais que l'utilisation du catalyseur C4 conforme à l'invention permet de limiter la formation de produits légers (coupe 150°C⁻).

**Tableau 1**

| Catalyseur | C1 | C2 | C3 | C4 |
|---|---|---|---|---|
| % poids coupe 150°150°C⁻ | 30 | 15 | 20 | 11 |
| % poids coupe 150°C⁺ | 70 | 85 | 80 | 89 |
| Température Limite de Filtrabilité / °C | -38 | -42 | -39 | -40 |

### Exemple 7 : Hydroisomérisation de la change paraffinique - production de base kérosène.

La charge paraffinique obtenue dans l'exemple 1 est hydroisomérisée sur les différents catalyseurs selon le protocole défini dans l'exemple 6, la température de test étant ici ajustée pour obtenir un point de disparition des cristaux de la coupe 150°C⁺ voisin de -40°C.

Les rendements en coupe 150°C⁻, coupe 150°C⁺ (obtenus par distillation simulée) de l'effluent ainsi que le point de disparition des cristaux (obtenu par la méthode ASTM D7153) de la coupe 150°C⁺ de l'effluent sont reportés pour l'ensemble des catalyseurs dans le tableau 2.

**Tableau 2**

| Catalyseur | C1 | C2 | C3 | C4 |
|---|---|---|---|---|
| % poids coupe 150°C⁻ | 40 | 23 | 30 | 20 |
| % poids coupe 150° C⁺ | 60 | 77 | 70 | 80 |
| Point de disparition des cristaux / °C | -40 | -42 | -41 | -41 |

On constate que pour l'ensemble des catalyseurs il est possible d'obtenir une base kérosène présentant d'excellentes propriétés de tenue à froid mais que l'utilisation du catalyseur C4 conforme à l'invention permet de limiter la formation de produits légers (coupe 150°C⁻).

## Revendications

1. Procédé de conversion d'une charge paraffinique ayant un nombre d'atomes de carbone compris entre 10 et 22, ladite charge paraffinique étant produite à partir de ressources renouvelables, à l'exclusion des charges paraffiniques obtenues par un procédé mettant en jeu une étape de valorisation par la voie Fischer-Tropsch, ledit procédé mettant en oeuvre un catalyseur comprenant au moins un métal hydro-déshydrogénant choisi dans le groupe formé par les métaux du groupe VIB et du groupe VIII de la classification périodique, pris seuls ou en mélange et un support comprenant au moins une zéolithe IZM-2 et au moins un liant, ledit procédé étant effectuée à une température comprise entre 150 et 500°C, à une pression comprise entre 0,1 MPa et 15 MPa, à une vitesse spatiale horaire comprise entre 0,1 et 10h⁻¹ et en présence d'une quantité totale d'hydrogène mélangée à la charge telle que le ratio hydrogène/charge soit compris entre 70 et 2000 Nm³/m³ de charge.

2. Procédé selon la revendication 1 dans lequel ladite charge paraffinique est produite à partir de ressources renouvelables choisies parmi les huiles végétales, les huiles d'algues ou algales, les huiles de poissons et les graisses d'origine végétale ou animale, ou des mélanges de telles charges.

3. Procédé selon l'une des revendications 1 à 2 dans lequel ledit procédé selon l'invention est un procédé d'hydroisomérisation.

4. Procédé selon l'une des revendications 1 à 3 dans lequel les éléments du groupe VIII sont choisis parmi le cobalt, le nickel, le platine et le palladium, pris seul ou en mélange.

5. Procédé selon la revendication 4 dans lequel la teneur en métal noble dudit catalyseur est comprise entre 0,01 et 5% en poids par rapport à la masse totale dudit catalyseur.

6. Procédé selon l'une des revendications 1 à 5 dans lequel les éléments du groupe VIB sont choisis parmi le tungstène et le molybdène, pris seuls ou en mélange.

7. Procédé selon l'une des revendications 1 à 4 et 6 dans lequel la teneur en métal du groupe VIB est comprise entre 5 et 40% en poids d'oxyde par rapport à la masse totale dudit catalyseur, et la teneur en métal non noble du groupe VIII, est comprise entre 0,5 et 10% en poids d'oxyde par rapport à la masse totale dudit catalyseur.

8. Procédé selon l'une des revendications 1 à 7 dans lequel ledit catalyseur comprend de 2 à 80% en poids de zéolithe IZM-2 par rapport à la masse totale dudit catalyseur.

9. Procédé selon l'une des revendications 1 à 7 dans lequel ledit liant est choisi dans le groupe formé par l'alumine, la silice, la silice-alumine, les argiles, l'oxyde de titane, l'oxyde de bore et la zircone, pris seuls ou en mélange.

10. Procédé selon la revendication 9 dans lequel ledit catalyseur comprend de 5 à 98% en poids de liant par rapport à la masse totale dudit catalyseur.

11. Procédé selon l'une des revendications 1 à 10 dans lequel ledit procédé est effectué à une température comprise entre 150°C et 450°C, à une pression comprise entre 0,2 et 15 MPa, à une vitesse volumique horaire comprise entre 0,2 et 7 h⁻¹ et en présence d'une quantité totale d'hydrogène mélangée à la charge telle que le ratio hydrogène/charge soit compris entre 100 et 1500 normaux m³ d'hydrogène par m³ de charge.

## Patentansprüche

1. Umwandlungsverfahren einer paraffinischen Charge, die eine Anzahl von Kohlenstoffatomen zwischen 10 und 22 hat, wobei die paraffinische Charge ausgehend von erneuerbaren Ressourcen erzeugt wird, mit Ausschluss der Paraffinchargen, die durch ein Verfahren erhalten werden, das einen Valorisationsschritt durch Fischer-Tropsch-Synthese umsetzt, wobei das Verfahren einen Katalysator eingesetzt, der mindestens ein hydrierendes-dehydrierendes Metall umfasst, das aus der Gruppe ausgewählt ist, die aus den Metallen der Gruppe VIB und der Gruppe VII des Periodensystems besteht, die allein oder im Gemisch genommen werden, und einen Träger, der mindestens einen Zeolith IZM-2 und mindestens ein Bindemittel umfasst, wobei das Verfahren bei einer Temperatur zwischen 150 und 500°C, bei einem Druck zwischen 0,1 MPa und 15 MPa bei einer räumlichen stündlichen Geschwindigkeit zwischen 0,1 und 10 h⁻¹ und in Gegenwart einer Gesamtmenge von Wasserstoff, die mit der Charge gemischt ist, derart ausgeführt wird, dass das Verhältnis Wasserstoff/Charge zwischen 70 und 2000 Nm³/m³ Charge liegt.

2. Verfahren nach Anspruch 1, bei dem die Paraffincharge ausgehend von erneuerbaren Ressourcen erzeugt wird, die aus den Pflanzenölen, den Algenölen oder Algenflora, den Fischölen und den Fetten pflanzlicher oder tierischer Herkunft oder Gemischen solcher Chargen ausgewählt sind.

3. Verfahren nach einem der Ansprüche 1 bis 2, wobei das erfindungsgemäße Verfahren ein Hydroisomerisationsverfahren ist.

4. Verfahren nach einem der Ansprüche 1 bis 3, wobei die Elemente der Gruppe VIII aus Kobalt, Nickel, Platin und Palladium allein oder im Gemisch ausgewählt sind.

5. Verfahren nach Anspruch 4, wobei der Gehalt an Edelmetall des Katalysators zwischen 0,01 und 5 Gew.-% in Bezug auf die Gesamtmasse des Katalysators liegt.

6. Verfahren nach einem der Ansprüche 1 bis 5, wobei die Elemente der Gruppe VIB aus Wolfram und Molybdän, allein oder im Gemisch genommen ausgewählt sind.

7. Verfahren nach einem der Ansprüche 1 bis 4 und 6, wobei der Gehalt an Metall der Gruppe VIB zwischen 5 und 40 Gew.-% Oxid in Bezug auf die Gesamtmasse des Katalysators liegt, und der Gehalt an Nichtedelmetall der Gruppe VIII zwischen 0,5 und 10 Gew.-% Oxid in Bezug auf die Gesamtmasse des Katalysators liegt.

8. Verfahren nach einem der Ansprüche 1 bis 7, wobei der Katalysator 2 bis 80 Gew.-% Zeolith IZM-2 in Bezug auf die Gesamtmasse des Katalysators umfasst.

9. Verfahren nach einem der Ansprüche 1 bis 7, wobei das Bindemittel aus der Gruppe ausgewählt ist, die aus Aluminiumoxid, Siliziumdioxid, Siliziumdioxid-Aluminiumoxid, Tonerden, Titanoxid, Boroxid und Zirkon allein oder im Gemisch genommen besteht ist.

10. Verfahren nach Anspruch 9, wobei der Katalysator 5 bis 98 Gew.-% Bindemittel in Bezug auf die Gesamtmasse des Katalysators umfasst.

11. Verfahren nach einem der Ansprüche 1 bis 10, wobei das Verfahren bei einer Temperatur zwischen 150 °C und 450 °C, bei einem Druck zwischen 0,2 und 15 MPa, bei einer stündlichen Volumengeschwindigkeit zwischen 0,2 und 7 h⁻¹ und in Gegenwart einer Gesamtmenge von Wasserstoff, die mit der Charge gemischt ist, derart ausgeführt wird, dass das Verhältnis Wasserstoff/Charge zwischen 100 und 1500 Norm-m³ Wasserstoff pro m³ Charge liegt.

## Claims

1. Process for conversion of a paraffinic feedstock that has a number of carbon atoms of between 10 and 22, whereby said paraffinic feedstock is produced starting from renewable resources, excluding paraffinic feedstocks that are obtained by a process that involves a stage for upgrading by the Fischer-Tropsch method, whereby said process uses a catalyst that comprises at least one hydrogenating-dehydrogenating metal that is selected from the group that is formed by the metals of group VIB and group VIII of the periodic table, taken by themselves or in a mixture, and a substrate comprising at least one IZM-2 zeolite and at least one binder, whereby said process is carried out at a temperature of between 150 and 500°C, at a pressure of between 0.1 MPa and 15 MPa, at an hourly volumetric flow rate of between 0.1 and 10 h⁻¹, and in the presence of a total quantity of hydrogen that is mixed with the feedstock such that the hydrogen/feedstock ratio is between 70 and 2,000 Nm³/m³ of feedstock.

2. Process according to claim 1, in which said paraffinic feedstock is produced from renewable resources that are selected from among vegetable oils, alga or algal oils, fish oils, and fats of vegetable or animal origin, or mixtures of such feedstocks.

3. Process according to one of Claims 1 to 2, in which said process according to the invention is a hydro-isomerization process.

4. Process according to one of Claims 1 to 3, in which the elements of group VIII are selected from among cobalt, nickel, platinum, and palladium, taken by itself or in a mixture.

5. Process according to Claim 4, in which the content of noble metal of said catalyst is between 0.01 and 5% by weight relative to the total mass of said catalyst.

6. Process according to one of Claims 1 to 5, in which the elements of group VIB are selected from among tungsten and molybdenum, taken by themselves or in a mixture.

7. Process according to one of Claims 1 to 4 and 6, in which the metal content of group VIB is between 5 and 40% by weight of oxide relative to the total mass of said catalyst, and the content of non-noble metal of group VIII is between 0.5 and 10% by weight of oxide relative to the total mass of said catalyst.

8. Process according to one of Claims 1 to 7, in which said catalyst comprises 2 to 80% by weight of IZM-2 zeolite relative to the total mass of said catalyst.

9. Process according to one of Claims 1 to 7, in which said binder is selected from the group that is formed by alumina, silica, silica-alumina, clays, titanium oxide, boron oxide, and zirconia, taken by themselves or in a mixture.

10. Process according to Claim 9, in which said catalyst comprises 5 to 98% by weight of binder relative to the total mass of said catalyst.

11. Process according to one of Claims 1 to 10, in which said process is carried out at a temperature of between 150°C and 450°C, at a pressure of between 0.2 and 15 MPa, at an hourly volumetric flow rate of between 0.2 and 7 h⁻¹, and in the presence of a total quantity of hydrogen mixed with the feedstock such that the hydrogen/feedstock ratio is between 100 and 1,500 normal m³ of hydrogen per m³ of feedstock.
